# EUROPEAN PATENT SPECIFICATION

(11) **EP 1 907 849 B1**
(45) Date of publication and mention of the grant of the patent: **26.08.2009**
(21) Application number: 06783860.7
(22) Date of filing: 28.07.2006
(51) Int. Cl.: G01N 33/543

(54) **SYSTEM FOR THE MANIPULATION OF MAGNETIC PARTICLES**
SYSTEM ZUR MANIPULATION MAGNETISCHER TEILCHEN
SYSTÈME DE MANIPULATION DE PARTICULES MAGNÉTIQUES

(30) Priority: 28.07.2005 EP 05076745
(43) Date of publication of application: 09.04.2008
(73) Proprietor: Nederlandse Organisatie voor Toegepast-Natuurwetenschappelijk Onderzoek TNO, 2628 VK Delft (NL)
(72) Inventor: KEIJZER, Paul, NL-2623 CL Delft (NL); HOEGAERTS, Christophe, Louis, Gabriel, NL-2521 ZJ Den Haag (NL)
(74) Representative: Hatzmann, Martin
(86) International application number: PCT/NL2006/000392
(87) International publication number: WO 2007/013805

(56) References cited:
- EP-A- 0 547 015
- US-A- 5 674 401
- RIDA A ET AL: "Planar coil-based microsystem for the long-range transport of magnetic beads" TRANSDUCERS '03. 12TH INTERNATIONAL CONFERENCE ON SOLID-STATE SENSORS, ACTUATORS AND MICROSYSTEMS. DIGEST OF TECHNICAL PAPERS (CAT. NO.03TH8664) IEEE PISCATAWAY, NJ, USA, vol. 1, 2003, pages 292-295 vol.1, XP002372612 ISBN: 0-7803-7731-1
- RIDA A ET AL: "Dynamics of magnetically retained supraparticle structures in a liquid flow" APPLIED PHYSICS LETTERS AIP USA, vol. 85, no. 21, 22 November 2004 (2004-11-22), pages 4986-4988, XP002372613 ISSN: 0003-6951

## Description

### Domain:

The inventions concerns a system for the manipulation of magnetic particles ("magnetic beads"). Magnetic particles are defined here as particles having good magnetic permeability, by which the particles can be attracted by a magnet, at a low magnetic remanence, so that the particles themselves do not become (permanently) magnetic.

### Background

The invention concerns a technology that is useful for generic sampling preparation for, for example, DNA analysis of micobiologic organisms, RNA extraction or the isolation of proteins.

For DNA analysis of microbiologic organisms 3 steps can be distinguished:
1. Sampling and preconcentration
2. Sampling preparation
3. DNA analysis

At the moment, a lot of research and development is taking place in the field of DNA analysis (for example DNA arrays), whereas the development of automatized systems for sampling preparation hardly takes place. The actions for sampling preparation are done manually by an analyst in the laboratory.

For a sampling preparation, the DNA needs to be isolated (for example 10 µl pure DNA in resolutive) from a concentrated rough sample (for example 200 µl aqueous solution with cells and contamination). For the sampling preparation, the following steps can be distinguished:
1. Lysing (breaking open of the cells, by which the DNA is released)
2. Washing (the separating of pure DNA and cells rests and other contaminations)
3. Eluting (the solution of the DNA into a resolutive)

It is, particularly for the steps 2 and 3, desirable to be able to immobilize and transport the DNA. For this, a possible method is the use of magnetic particles in combination with magnets.

Magnetic particles have a DNA binding surface (for example glass) and are furthermore for example ferriferous. At the market, there is a fairly wide range of magnetic particles available (see for example www.agowa.de/struktur/magneticbasis.html or www.chemagen.de/uk/mDva/features/features.html). Releasing DNA will be bound by the surface of the particles. The particles can then be immobilized by the analyst by means of magnets, after which the washing and eluting can take place.

It is necessary for an automatized system to make switchable the magnet used for the immobilization of the particles, by which the particles can not be attracted by the magnet in one switching status and can be attracted in a second switching status. Known configurations comprise for this:
- A switchable electromagnet that, used for a similar purpose, is known from DE19955169. By an electrical current through a coil around a coil core a flux is generated in an air gap. Switching off the current thus means the switching off of that flux.
- In a moveable permanent magnet, for example a revolving permanent magnet in a magnetic loop, known from W02005/005049. The permanent magnet and the air gap for the samples to be analyzed are put together in a soft iron loop. When the north-south axis of the permanent magnet is in line with the magnetic path through the soft iron, there is a magnetic field in the air gap. If the permanent magnet is turned 90° degrees, the flux in the air gap will become nearly or entirely equal to zero.

It is noted that European patent publication EP 0 547 015 and American patent publication US 5 674 401 disclose an apparatus for detecting and monitoring lubricating oil, respectively. The apparatus disclosed in EP '015 and US '401 includes a permanent magnet, an electromagnet and a switching means for changing the polarity of the electromagnet.

It is further noted that the articles "planar coil-based microsystem for the long-rang transport of magnetic beads" in Transducers '03 by A. Rida et al. and "Dynamics of magnetically retained supraparticle structure in a liquid flow" in Applied physics letters by A. Rida et al. disclose an apparatus including a permanent magnet and an electromagnet.

The disadvantages of the known configurations are:
1. Conventional electromagnets use electricity during the full time of operation, thus producing thermal dissipation. This can notably in a microsystem (less
2. heat exchanging surface) lead to problems due to undesirable increase in temperature.
3. Conventional electromagnets are large in volume in order to avoid that they absorb too much power and/or become to hot.
4. Moving parts.
5. For mechanically switchable magnets (by electrical motor or manually), the time to switch the magnet can amount to tens of seconds.

### Summary

The invention relates to a system according to claim 1. Further, the invention relates to a method according to claim 6.

The hereinafter presented new system for the manipulation of magnetic particles by means of a switchable magnetic flux source comprises a first magnetic flux source, comprising one or more permanent magnets and a second magnetic flux source, comprising one or more modules, each with a coil equipped with a coil core through which coil an electrical current can flow which is controlled by switching means. The switching means have been preferably adapted to give, in a first switching status - in which a minimal flux flows through the particles - such a direction to the electrical current that the flux of the first flux source and the flux of the second flux source get an opposite direction, by which then the resulting flux is minimal, preferably zero or nearly zero.

In this configuration the switchable magnetic flux source enters into a second switching status - in which the flux through the particles is maximal and in which the particles can be attracted by the switchable magnetic flux source - as soon as the electrical current through the coil is interrupted. At least, if the coil core has such a constitution - for example when a coil core is made of soft iron - that the remanent magnetism in the coil core is minimal. In this configuration - with e.g. a soft iron core - it is necessary that the electrical current flows uninterruptedly through the coil as long as the first switching status continues. As soon as the current is interrupted, the second flux disappears and the resulting flux becomes mainly equal to the flux generated by the permanent magnets.

According to an aspect of the invention, however, use is made of a system as described above, in which the coil core however has such a constitution that, as a result of remanent magnetism, the second flux source remains substantially active, also after the interruption of the electrical current. In this configuration, with, in this case, a core of magnetizable material it is not necessary that the electrical current flows uninterruptedly through the coil as long as the firs switching status continues. As soon as the current is interrupted, the second flux mainly is preserved and the resulting flux approximately equal to zero, assuming that the current direction and current intensity are such that the first and second flux source are equally strong, but oppositely directed. The first switching status thus continues in this preferred configuration after the interruption of the coil current. The advantage of this configuration is that the electrical current is exclusively needed for magnetizing the coil core. As soon as this is magnetized, the current is interrupted, but the (remanent) magnetism remains. The electrical load and the thermal dissipation are in this embodiment considerably slighter than in the preceding configuration. As a consequence, the system can advantageously be applied for sampling preparation of DNA structures, as these structures are relatively sensitive to heat. Further, due to the reduced thermal dissipation, the system can advantageously be implemented in a relatively small housing, so that a more manageable unit is obtained.

In order to put the system back into the second switching status (with maximal flux), it is necessary to reverse the polarity of the second flux source, by which the first, permanent flux source will no longer be opposed by the second, semi-permanent or bi-stable flux source. It is possible to adjust the current magnitude , the current direction and the time in such a way that the current brings the remanent magnetism back to zero level, by which the net flux through the switchable magnetic flux source is equal to the flux generated by the permanent flux source. By e.g. choosing the current intensity higher, the second flux source in the second switching status can get a magnetic field that is unequal to zero and that - different from during the first switching status - even cooperates with the field of the permanent first flux source, by which the resulting flux through the system is larger than the flux generated solely by the first flux source.

For this reason, the switching means are preferably arranged to give in a first switching status such a direction and power to the said electrical current that after interruption of that electrical current the flux of the second flux source generated by the remanent magnetism is mainly equal to the flux of the first flux source, by which the resulting flux through the switchable magnetic flux source is zero or nearly zero.

In the system as presented above, the first flux source and the second flux source can be configured either parallel to or in series with one another.

In the said preferred configuration of the invention, only - as said - an electrical power is needed during switching from the first to the second switching status and vice versa, but not during the first or the second switching status themselves. By this a smaller system (device, apparatus) can be realized which, besides, dissipates little heat. The inherent weight savings improve the portability. Finally, the switching speed is relatively high (about 0.2 seconds), improving the whole process.

### Exemplary embodiment

Figure 1 shows the exemplary embodiment of the system according to the invention.
Figure 2 shows a detail of the exemplary embodiment showed in figure 1.
Figure 3 and 4 show the flux flow in respectively the first and second switching status.

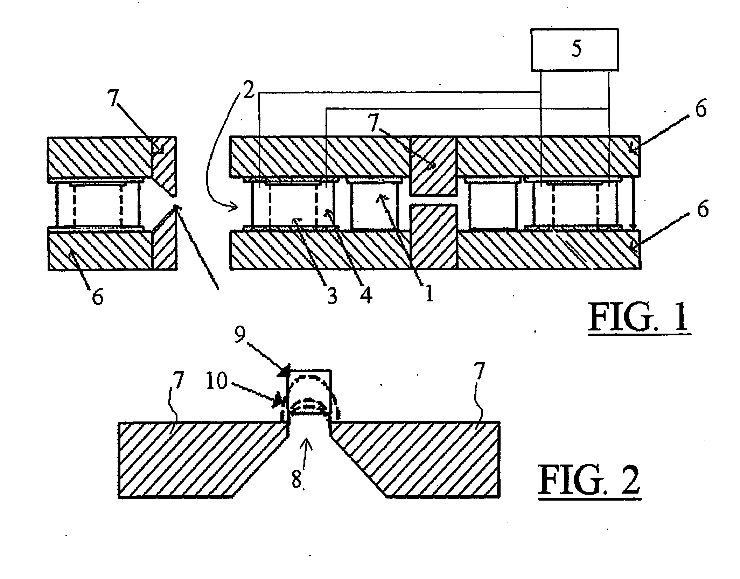

The exemplary embodiment of a system for the manipulation of magnetic particles by means of a switchable magnetic flux source, in shown figure 1, comprises a first magnetic flux source, formed by permanent magnets 1 and a second magnetic flux source, formed by modules 2, each with an electrical coil 4 equipped with a coil core 3. The coils 4 can be run through by an electrical current controlled by a switching module 5.

The first flux source and the second flux source are configured parallel to each other by means of two soft iron yoke members 6 of which one lies on top and one on the underside, against the permanent magnets 1 and the coil cores 3. The first and the second flux source could also be configured in series with one another by the permanent magnets 1 and coil cores 3, not as shown in figure 1, but by putting them as a pair in line with each other and by connecting each of those pairs by means of the soft iron yoke members.

To the yoke members 6 a set of soft iron bridge members 7 is connected which - except a narrow air gap 8 - form the return path (bridge member) for the (net) magnetic flux generated by the permanent magnets 1 and the modules 2. In the air gap 8 between the extremities of the two bridge members, preferably ending in a point, a strong and strongly divergent magnetic field 10 can be found. This strong, divergent field 10 is pre-eminently appropriate in a room 9 in the proximity of the air gap 8 to drive magnetic particles (not shown) into the direction of the air gap 8 and thus to immobilize them.

The switching module 5 is arranged to give, in a first switching status, the electrical current through the coil cores 4 such a direction that the flux of the first flux source, the permanent magnets 1, and the flux of the second flux source, the modules 2, have opposite directions. As said, the coil cores 3 have such a constitution that, as a result of remanent magnetism, the second flux source remains substantially active after the electrical current has been interrupted; in other words the coil cores 3 work in this case as (semi-)permanent magnets, of which the direction and magnitude of the flux is set by the electrical current which - controlled by the module 5 - flowed for the last time through the coils 4.

The switching module 5 is fit for briefly generating an electrical current through the coils 4 in the first switching status, with such a direction and magnitude that after interruption of that electrical current the flux in the coil cores 3, caused by the remanent magnetism, is mainly equal to the flux generated by the permanent magnets 1, in such a way that the resulting flux through the whole systems (yoke members 6, bridge members 7 and air gap 8) is about zero.

By means of the electrical current through the coils 4, controlled by the switching module 5, the polarity of part of the magnets - viz. the semi-permanent magnets 3 - can be reversed. The contributions of the different magnets - the permanent magnets 1 and the semi-permanent magnets 3 - to the total magnetic field - in the first switching status - will neutralize one another or - in the second switching status - do not neutralize one another (and do or don't reinforce one another). In this way, in the first switching status the magnetic particles can freely move in the room 9, whereas they are immobilized in the second switching status.

The room 9 has for example dimensions of 3 x 10 x 3 mm. The air gap 8 then is e.g. 3 mm long and 10 mm large. Because the soft iron yoke members 6 have a mutual distance of 11 mm and the air gap 8 is only 3 mm long, the magnetic field will be concentrated in the air gap. The bridge members 7 are preferably placed in the middle of the construction in order to have a rest field that is as low as possible in 'switched off' position (at complete symmetry).

Thus, the semi-permanent magnets 3 have three states, viz. a NZ polarity, a ZN polarity and a neutral state. In order to perform a transformation from the neutral polarity to a NZ or ZN polarity or vice versa, a magnetization operation or a demagnetization operation, respectively, is executed, using electric control currents having a temporal amplitude and direction behaviour, such that, as a result of remanent magnetism, the second magnetic flux source remain substantially in a predetermined switching state, after interruption of the electrical current, as described above. In particular, the semi-permantent magnets are brought into a NZ polarity, ZN polarity or neutral status. Similarly, to change a NZ polarity into a ZN polarity or vice versa, a polarization operation is performed, also using such electric control currents.

As an example, in the first switching status of the switching module 5, the semi-permanent magnets 3 have been magnetized, e.g. having a NZ polarity or ZN polarity, counteracting the magnetic flux of the permanent magnets 1 in such a way that the resulting flux is about zero. In the second switching status, the semi-permanent magnets 3 have been polarized, e.g. having a ZN polarity or NZ polarity, respectively, amplifying the magnetic flux of the permanent magnets 1.

In an alternative embodiment, the first switching status of the switching module 5 coincides with the first switching status described above. However, in the second switching status of the alternative embodiment, the semi-permanent magnets 3 have been demagnetized, so that the semi-permanent magnets 3 do not substantially generate a magnetic flux in this status. As a consequence, the magnetic flux of the permanent magnets 1 substantially constitutes the total magnetic flux generated by the system.

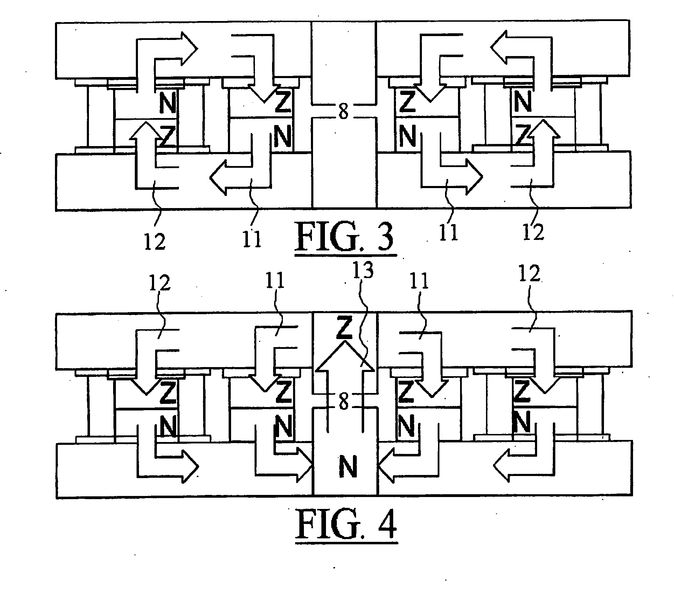

Finally, the figures 3 and 4 show the flux flow in respectively the first switching status (figure 3) and the second switching status (figure 4). The permanent magnets 1 generate in both cases a permanent flux 11, the semi-permanent magnets 3 in both cases a flux 12, which is however in figure 3 (first switching status) opposite to the flux 11, resulting in a flux 13 of about zero through the air gap 8. In the second switching status (figure 4) the flux 12 has the same direction as the permanent flux 11, resulting into a maximal flux 13 through the air gap 8.

Preferably, the magnetic fields generated by the magnetic flux source are relatively high, e.g. more than 800 mT, to immobilize the particles in the DNA structure. In order to magnetize, demagnetize or polarize the magnetizable material in coil cores of the second magnetic flux source, relatively high peak values of the magnetic field are generated, up to e.g. 4T.

## Claims

1. System for manipulation of magnetic particles by means of a switchable magnetic flux source, comprising a first magnetic flux source, comprising one or more permanent magnets (1), and a second magnetic flux source, comprising one or more modules (2), each with an electrical coil (4) equipped with a coil core (3), which can be run through by an electrical current, controlled by switching means (5), wherein the coil core comprises magnetizable material and has such a constitution that, as a result of remanent magnetism, the second flux source remains substantially in a predetermined switching state, after interruption of the electrical current having a temporal amplitude and direction behaviour.

2. System according to claim 1, where the switching means are arranged to give, in a first switching status, such a direction to the electrical current that the flux of the first flux source and the flux of the second flux source have opposite directions.

3. System according to claim 1 or 2, wherein the switching means are arranged to give, in the first switching status, such a direction and magnitude to the electrical current that after interruption of that electrical current the flux of the second flux source, generated by the remanent magnetism, is mainly equal to the flux of the first flux source, in such a way that the resulting flux through the switchable magnetic flux source is zero or nearly zero.

4. System according to claim 1, wherein the first flux source and the second flux source are configured parallel to each other in the switchable magnetic flux source.

5. System according to claim 1, wherein the first flux source and the second flux source are configured in series with one another in the switchable magnetic flux source.

6. Method for manipulation of magnetic particles by means of a switchable magnetic flux source, comprising a first magnetic flux source, comprising one or more permanent magnets (1), and a second magnetic flux source, comprising one or more modules (2), each with an electrical coil (4) equipped with a coil core (3) comprising magnetizable material, wherein the method comprises the step of applying through the electrical coil an electrical current having a temporal amplitude and direction behaviour such that, as a result of remanent magnetism, the second flux source remains substantially in a predetermined switching state, after interruption of the electrical current.

## Patentansprüche

1. System zur Manipulation von magnetischen Partikeln mittels einer schaltbaren Magnetflussquelle, mit einer ersten Magnetflussquelle, die einen oder mehrere Permanentmagnete (1) enthält, und mit einer zweiten Magnetflussquelle, die ein oder mehrere Module (2) enthält, wovon jedes eine mit einem Spulenkern (3) versehene elektrische Spule (4) aufweist, die von einem elektrischen Strom durchströmt werden kann, der durch Schalteinrichtungen (5) gesteuert wird, wobei der Spulenkern magnetisierbares Material enthält und eine solche Ausgestaltung hat, dass, als eine Folge von remanentem Magnetismus, die zweite Flussquelle nach Unterbrechung des elektrischen Stroms, der eine temporäre Amplitude und Richtungsverhalten hat, im Wesentlichen in einem vorbestimmten Schaltzustand verbleibt.

2. System nach Anspruch 1, bei dem die Schalteinrichtungen ausgestaltet sind, um, in einem ersten Schaltzustand, dem elektrischen Strom eine solche Richtung zu verleihen, dass der Fluss der ersten Flussquelle und der Fluss der zweiten Flussquelle entgegen gesetzte Richtungen haben.

3. System nach Anspruch 1 oder 2, bei dem die Schalteinrichtungen ausgestaltet sind, um, in dem ersten Schaltzustand, dem elektrischen Strom eine solche Richtung und Höhe zu verleihen, dass, nach Unterbrechung dieses elektrischen Stroms, der Fluss der zweiten Flussquelle, erzeugt durch den remanenten Magnetismus, im Wesentlichen gleich dem Fluss der ersten Flussquelle ist, und zwar in einer solchen Weise, dass der resultierende Fluss durch die schaltbare Magnetflussquelle Null oder nahezu Null ist.

4. System nach Anspruch 1, bei dem die erste Flussquelle und die zweite Flussquelle in der schaltbaren Magnetflussquelle parallel zueinander konfiguriert sind.

5. System nach Anspruch 1, bei dem die erste Flussquelle und die zweite Flussquelle in der schaltbaren Magnetflussquelle in Reihe zueinander konfiguriert sind.

6. Verfahren zur Manipulation von magnetischen Partikeln mittels einer schaltbaren Magnetflussquelle, mit einer ersten Magnetflussquelle, die einen oder mehrere Permanentmagnete (1) enthält, und mit einer zweiten Magnetflussquelle, die ein oder mehrere Module (2) enthält, wovon jedes eine elektrische Spule (4) aufweist, die mit einem Spulenkern (3) versehen ist, der magnetisierbares Material enthält, wobei das Verfahren die Schritte umfasst: Bewirken, dass durch die elektrische Spule ein elektrischer Strom fließt, der eine temporäre Amplitude und Richtungsverhalten hat, so dass, als eine Folge von remanentem Magnetismus, die zweite Flussquelle nach Unterbrechung des elektrischen Stroms im Wesentlichen in einem vorbestimmten Schaltzustand verbleibt.

## Revendications

1. Système de manipulation de particules magnétiques au moyen d'une source de flux magnétique commutable, comprenant un ou plusieurs aimants permanents (1) et une seconde source de flux magnétique comprenant un ou plusieurs modules (2) comportant chacun une bobine électrique (4) équipée d'un noyau de bobine (3) susceptible d'être traversé par un courant électrique commandé par des moyens de commutation (5), dans lequel le noyau de bobine comprend un matériau magnétisable et présente une constitution telle que sous l'effet du magnétisme rémanent, la seconde source de flux reste sensiblement dans un état de commutation prédéterminé après interruption du courant électrique présentant un comportement temporel en termes de sens et d'amplitude.

2. Système selon la revendication 1, **caractérisé en ce que** les moyens de commutation sont agencés pour, dans un premier état de commutation, donner au courant électrique un sens tel que le flux de la première source de flux et le flux de la seconde source de flux sont de sens opposés.

3. Système selon la revendication 1 ou la revendication 2, **caractérisé en ce que** les moyens de commutation sont agencés pour donner au courant électrique, dans le premier état de commutation, un sens et une amplitude tels que, après interruption dudit courant électrique, le flux de la seconde source, généré par le magnétisme rémanent, est principalement égal au flux de la première source de flux, de telle sorte que le flux résultant qui traverse la source de flux magnétique commutable est nul ou quasi nul.

4. Système selon la revendication 1, **caractérisé en ce que** la première source de flux et la seconde source de flux sont configurées en parallèle dans la source de flux magnétique commutable.

5. Système selon la revendication 1, **caractérisé en ce que** la première source de flux et la seconde source de flux sont configurées en série dans la source de flux magnétique commutable.

6. Procédé de manipulation de particules magnétiques au moyen d'une source de flux magnétique commutable comprenant une première source de flux magnétique comportant un ou plusieurs aimants permanents (1), et une seconde source de flux magnétique comprenant un ou plusieurs modules (2) comportant chacun une bobine électrique (4) équipée d'un noyau de bobine (3) comprenant un matériau magnétisable, **caractérisé en ce que** ledit procédé comprend l'étape consistant à faire traverser la bobine électrique par un courant électrique ayant un comportement temporel en termes d'amplitude et de sens tel que du fait du magnétisme rémanent, la seconde source de flux reste sensiblement dans un état de commutation prédéterminé après interruption du courant électrique.
